# DEMANDE DE BREVET EUROPEEN

(11) **EP 0 806 738 A1**
(43) Date de publication de la demande: **12.11.1997**
(21) Numéro de dépôt: 97401040.7
(22) Date de dépôt: 07.05.1997
(51) Int. Cl.: G06F 19/00

(54) **Dispositif à réseaux de neurones pour déterminer la quantité d'une substance à administrer à un patient**

(30) Priorité: 07.05.1996 FR 9605704
(71) Demandeur: Société D'Etudes Techniques - S E T, 75010 Paris (FR)
(72) Inventeur: Perez Laurent, 94130 Nogent-sur-Marne (FR); Tarlier Pascal, 77100 Nanteuil les Meaux (FR)
(74) Mandataire: Leszczynski, André

(57) **Abrégé**

Dispositif pour déterminer la quantité d'une substance à administrer à un patient en vue de modifier un état physiologique de ce dernier, susceptible de varier en réponse à l'administration de ladite substance, et se rapprocher d'un état physiologique cible.

Il comporte plusieurs réseaux de neurones ayant été entraînés, à partir d'un ensemble de données représentatives de cas expérimentaux et/ou cliniques, pour fournir une indication de la quantité préconisée de ladite substance à administrer en fonction dudit état physiologique cible, de paramètres propres au patient considéré et de la connaissance d'au moins un état physiologique antérieur de ce dernier, chaque réseau de neurones étant en outre spécifique à une période prédéterminée du traitement du patient.

## Description

La présente invention concerne un dispositif pour déterminer la quantité d'une substance à administrer à un patient en vue de modifier un état physiologique de ce dernier, susceptible de varier en réponse à l'administration de ladite substance, et se rapprocher d'un état physiologique cible.

L'invention concerne plus particulièrement mais non exclusivement l'insulinothérapie, c'est-à-dire l'administration d'insuline ou tout produit équivalent à un patient souffrant de diabète, pour contrôler sa glycémie.

L'insuline est habituellement administrée par voie intraveineuse sous forme d'une perfusion continue.

Il est difficile de déterminer exactement le débit d'insuline perfusée qui permet d'atteindre une glycémie cible car de nombreux facteurs interviennent dans l'évolution de la glycémie, tels qu'un repas récent, l'activité physique du patient, son état nerveux, etc...

Le débit d'insuline perfusée est déterminé plus ou moins empiriquement par des équipes médicales spécialisées en fonction de mesures régulières de la glycémie et de l'expérience acquise dans ce domaine.

Compte-tenu de la difficulté de réguler la glycémie chez un patient, les équipes médicales compétentes en insulinothérapie sont relativement peu nombreuses et il existe un besoin pour disposer d'un dispositif qui permettrait d'assister une équipe médicale non spécialisée dans le traitement par insulinothérapie de malades souffrant de diabète.

L'invention a ainsi pour objet un dispositif pour déterminer la quantité d'une substance à administrer à un patient en vue de modifier un état physiologique de ce dernier, susceptible de varier en réponse à l'administration de ladite substance, et se rapprocher d'un état physiologique cible.

Selon l'invention, ce dispositif comporte plusieurs réseaux de neurones ayant été entraînés, à partir d'un ensemble de données représentatives de cas expérimentaux et/ou cliniques, pour fournir une indication de la quantité préconisée de ladite substance à administrer en fonction dudit état physiologique cible, de paramètres propres au patient considéré et de la connaissance d'au moins un état physiologique antérieur de ce dernier, chaque réseau de neurones étant en outre spécifique à une période prédéterminée du traitement du patient.

Dans un cas particulier non limitatif de l'invention, ledit état physiologique est la glycémie et ladite substance à administrer est de l'insuline ou un produit équivalent.

Ainsi, on dispose grâce à l'invention d'un dispositif capable de préconiser la quantité d'insuline à administrer pour atteindre une glycémie cible, qui peut être utilisé par un personnel n'ayant reçu aucune formation particulière en insulinothérapie, voire par le patient lui-même ou qui pourrait même réguler la glycémie de manière autonome.

Dans une réalisation particulière de l'invention, chaque réseau de neurones est entraîné en présentant au réseau une succession de patrons d'entrée comportant chacun parmi ses composantes une valeur représentative de la glycémie mesurée dans un cas expérimental et/ou clinique et en imposant au réseau pour chaque cas comme patron de sortie une valeur représentative du débit d'insuline administré.

En variante, on entraîne chaque réseau de neurones en présentant au réseau une succession de patrons d'entrée comportant chacun parmi ses composantes une valeur représentative du débit d'insuline administré dans un cas expérimental et/ou clinique et en imposant au réseau pour chaque cas comme patron de sortie une valeur représentative de la glycémie mesurée ayant conduit à l'administration dudit débit d'insuline.

Dans un exemple particulier de mise en oeuvre de l'invention, un nombre N de mesures de la glycémie étant effectuées chaque jour, le dispositif comporte un premier ensemble de réseaux de neurones utilisés pour préconiser un débit d'insuline à administrer durant chaque période s'étendant entre deux mesures consécutives à partir de la deuxième mesure de glycémie jusqu'à la Nième mesure de glycémie, et un deuxième ensemble de réseaux de neurones utilisés pour préconiser un débit d'insuline à administrer pour chaque période ultérieure s'étendant entre deux mesures consécutives de la glycémie.

Selon une réalisation particulière de l'invention, le dispositif comporte en outre un dispositif d'injection.

D'autres caractéristiques et avantages de la présente invention apparaîtront à la lecture de la description détaillée qui va suivre, d'un exemple de réalisation non limitatif de l'invention, et à l'examen du dessin annexé sur lequel :
- la figure 1 représente de façon schématique un dispositif conforme à un exemple de réalisation de l'invention,
- la figure 2 représente de façon schématique l'organisation générale des moyens de calcul utilisés, et
- les figures 3 et 4 sont deux chronogrammes illustrant différentes phases du fonctionnement du dispositif.

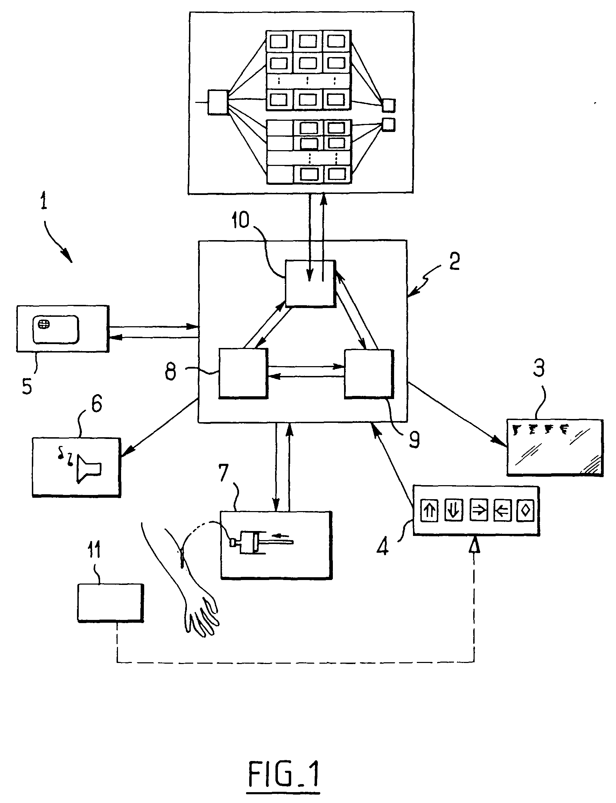

On a représenté sur la figure 1 un dispositif 1 conforme à un exemple de réalisation de l'invention, qui se présente avantageusement sous la forme d'un appareil portatif alimenté par piles.

Ce dispositif 1 comporte une unité centrale 2 et un certain nombre de périphériques reliés à cette dernière, dont un afficheur 3, un clavier 4, un lecteur-enregistreur de carte à puce 5, une alarme sonore 6 et un dispositif d'injection 7, représentés schématiquement.

On a désigné sous la référence 11 des moyens de mesure de la glycémie du patient.

L'unité centrale 2 comporte une horloge interne, deux microcontrôleurs 8,9 redondants pour la gestion des périphériques et un microcontrôleur 10 programmé pour préconiser, comme cela sera précisé dans la suite, une dose d'insuline à administrer.

Les microcontrôleurs 8,9 et 10 sont agencés en boucle pour échanger des informations et se contrôler mutuellement.

Le dispositif 1 permet d'assister un personnel non spécialisé en insulinothérapie dans le choix de la dose d'insuline à administrer à un patient en vue de contrôler sa glycémie.

On considère pour la suite que chaque jour dix mesures de la glycémie sont effectuées, par prélèvement de sang capillaire, aux heures suivantes :
1h 30, 4 h, 7 h 30, 9 h, 12 h, 13 h 30, 16 h, 19 h, 20 h et 23 h.

On suppose que les repas sont pris régulièrement chaque jour aux heures suivantes :
7 h 30, 12 h et 19 h.

On désignera par "périodes prandiales" les tranches horaires commençant à l'instant de la mesure de la glycémie coïncidant avec le début d'un repas et se terminant à l'instant de la mesure suivante de la glycémie.

Dans l'exemple considéré, les périodes prandiales correspondent donc aux tranches horaires suivantes :
de 7 h 30 à 9 h, de 12 h à 13 h 30 et de 19 h à 20 h.

Le dispositif d'injection 7 comporte un mécanisme pour avancer le piston d'une cartouche d'insuline, et l'on contrôle le débit d'insuline administré par perfusion intraveineuse au patient en agissant sur la vitesse d'avancement du piston.

On désignera dans la suite par "débit de base" un débit d'insuline administré par le dispositif d'injection 7 hors des périodes prandiales.

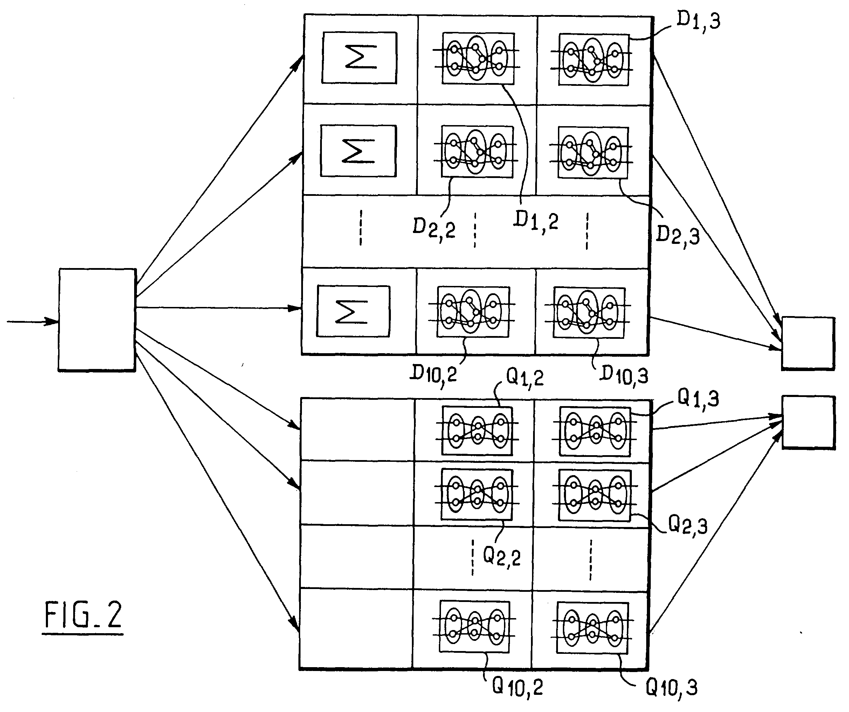

On a représenté de façon schématique sur la figure 2 l'organisation générale des moyens de calcul utilisés.

D'une façon générale, on distingue trois phases successives dans le traitement du patient, la première phase s'étendant entre les première et deuxième mesures de glycémie, la deuxième phase s'étendant entre les deuxième et dixième mesures de glycémie, et la troisième phase commençant à partir de la onzième mesure de glycémie.

Lors de la première phase, on calcule le débit d'insuline préconisé sans faire intervenir de réseaux de neurones, comme cela sera précisé dans la suite.

Pour les deuxième et troisième phases, on calcule le débit d'insuline préconisé en faisant intervenir à chaque fois un réseau de neurones.

On distingue lors de la deuxième phase neuf tranches horaires successives s'étendant chacune entre deux mesures de glycémie consécutives.

Pour chacune de ces tranches horaires on calcule le débit d'insuline préconisé au moyen d'un réseau de neurones spécifique.

Etant donné qu'il y a dix mesures de glycémie effectuées chaque jour, il faut prévoir dix réseaux de neurones pour les tranches horaires respectives débutant à chacune de ces mesures de glycémie.

On a référencé de façon schématique sur la figure 2 D_{1,2},D_{2,2},...,D_{10,2} ces dix réseaux de neurones.

Le réseau de neurones D_{1,2} est associé à la tranche horaire commençant à partir de la première mesure de la glycémie effectuée chaque jour, etc ...

On divise la troisième phase en cycles de dix tranches horaires successives s'étendant chacune entre deux mesures de la glycémie consécutives.

Pour chacune des dix tranches horaires d'un cycle, on calcule le débit d'insuline préconisé au moyen d'un réseau de neurones spécifique.

On utilise ainsi cycliquement les mêmes dix réseaux de neurones pour calculer le débit d'insuline préconisé lors de la troisième phase, que l'on a respectivement référencés D_{1,3},D_{2,3},..., D_{10,3} sur la figure 2.

Le réseau de neurones D_{1,3} est associé à la tranche horaire commençant à partir de la première mesure de glycémie effectuée chaque jour, etc...

Lors de la première phase du traitement, on distingue quatre cas selon que l'on se situe pendant l'une des trois périodes prandiales ou non.

Pour chacun des trois cas correspondant à une période prandiale, on calcule le débit d'insuline préconisé par une combinaison linéaire de quatre paramètres qui sont :
- le rapport du poids du patient au carré de sa taille,
- la glycémie mesurée,
- l'âge du patient,
- le poids du patient.

Pour le dernier cas, correspondant à une mesure de glycémie qui n'est pas effectuée au début d'un repas, on calcule le débit d'insuline préconisé par une combinaison linéaire de cinq paramètres qui sont :
- le rapport du poids du patient au carré de sa taille,
- la glycémie mesurée,
- l'âge du patient,
- le poids du patient et
- la glycémie cible.

Lors de la deuxième phase du traitement, on détermine le débit d'insuline préconisé en utilisant successivement neuf des dix réseaux de neurones D_{1,2},D_{2,2,}...,D_{10,2}.

On soumet au réseau de neurones utilisé un patron d'entrée ayant pour composantes :
- la dernière mesure de glycémie,
- l'avant-dernière mesure de glycémie,
- un indicateur booléen concernant une prise de sucre antérieure,
- un indicateur booléen concernant une injection antérieure ponctuelle d'insuline,
- le débit d'insuline administrée après l'avant-dernière mesure de glycémie,
- la valeur moyenne de tous les débits de base depuis le début du traitement et jusqu'à l'avant-dernière mesure de glycémie,
- le rapport du poids du patient au carré de sa taille
- son âge, et
- son poids.

Lors de la troisième phase du traitement, on utilise chacun des dix réseaux de neurones D_{1,3},D_{2,3},..., D_{10,3} en présentant au réseau sélectionné un patron d'entrée ayant pour composantes :
- la dernière mesure de glycémie,
- l'avant-dernière mesure de glycémie,
- l'antépénultième mesure de glycémie,
- un indicateur booléen concernant une prise de sucre antérieure,
- un indicateur booléen concernant une injection antérieure ponctuelle d'insuline,
- la glycémie mesurée la veille à la même heure,
- la glycémie mesurée la veille lors de la mesure suivant celle effectuée la veille à la même heure,
- un coefficient d'insulino-résistance,
- le débit d'insuline de la veille à la même heure,
- la valeur moyenne de tous les débits de base depuis le début du traitement,
- le débit d'insuline précédent, et
- l'antépénultien débit d'insuline.

Le coefficient d'insulino-résistance correspond à l'écart entre le débit d'insuline la veille à la même heure et le produit d'une valeur prédéterminée de débit d'insuline pour la tranche horaire considérée par le coefficient de régression linéaire entre tous les débits de base depuis le début du traitement et les valeurs prédéterminées de débit d'insuline pour les tranches horaires correspondantes.

A titre indicatif, les valeurs prédéterminées de débit d'insuline sont pour les dix tranches horaires respectivement associées aux dix mesures de glycémie effectuées périodiquement, à une constante multiplicative près :
0,8 ; 0,8 ; 1,2 ; 1,2 ; 1,2 ; 1,6 ; 1,6 ; 1,6 ; 1,3 ; 1,3

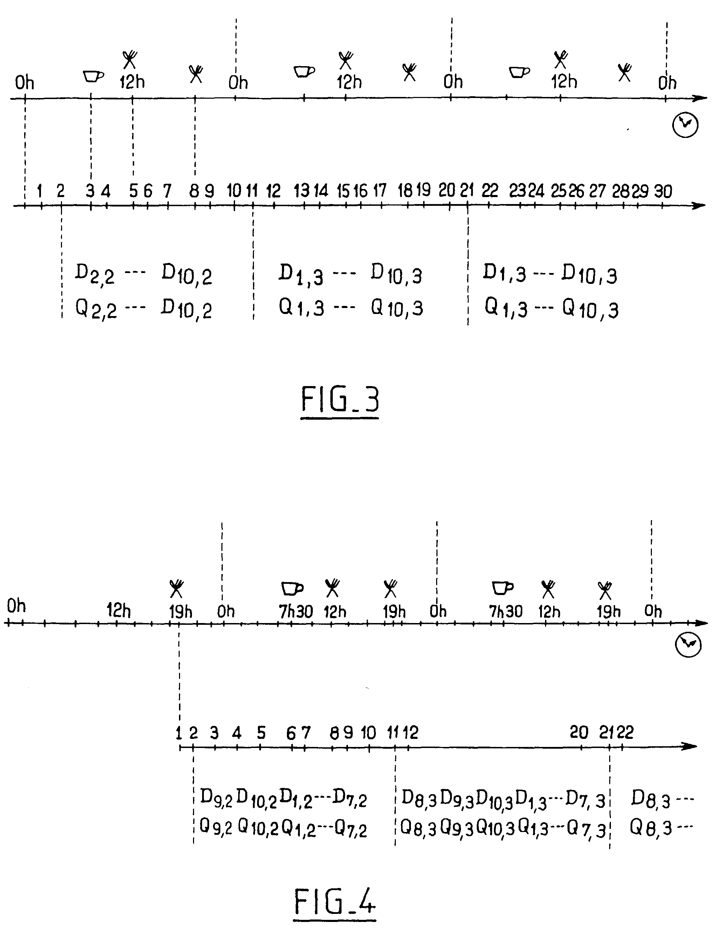

On a représenté sur les figures 3 et 4 deux exemples de chronogrammes sur lesquels on a reporté les différentes phases du traitement, avec sur chacune des figures une échelle inférieure précisant le nombre de mesures de glycémie effectuées depuis le début du traitement.

On remarquera que, dans l'exemple de la figure 3, la première phase du traitement ne correspond pas à une période prandiale, que la deuxième phase du traitement débute lors de la mesure de glycémie effectuée à 4 h du matin et que la troisième phase du traitement débute lors de la mesure de glycémie effectuée à 1 h 30 du matin.

Ainsi, lors de la deuxième phase, on utilise successivement les réseaux de neurones D_{2,2},..., D_{10,2} et lors de la troisième phase on utilise cycliquement les réseaux de neurones D_{1,3},..., D_{10,3}.

On remarquera à l'examen de la figure 4 que la première phase du traitement correspond dans l'exemple considéré à une période prandiale, que la deuxième phase du traitement débute lors de la mesure de glycémie effectuée à 20 h et que la troisième phase du traitement débute lors de la mesure de glycémie effectuée à 19 h le surlendemain du début du traitement.

Ainsi, lors de la deuxième phase, on utilise successivement les réseaux de neurones D_{9,2}, D_{10,2}, D_{1,2},..., D_{7,2}, et lors de la troisième phase on utilise cycliquement les réseaux de neurones D_{8,3}, D_{9,3}, D_{10,3}, D_{1,3},..., D_{7,3}.

Les réseaux de neurones D_{1,2},..., D_{10,2} et D_{1,3},..., D_{10,3} sont tous du type connu en soi perceptron multicouche totalement connecté avec une couche cachée et à fonction de transfert sigmoïde.

Le nombre de cellules d'entrée est déterminé par le nombre de composantes du patron d'entrée, le nombre de cellules cachées est dans l'exemple décrit égal à 3, et le nombre de cellules de sortie est égal à 1.

L'apprentissage de chaque réseau de neurones se fait à partir d'une base de données correspondant à des cas expérimentaux et/ou cliniques en présentant au réseau une succession de patrons d'entrée dont les composantes sont tirées desdits cas expérimentaux et/ou cliniques et en imposant comme patron de sortie le débit d'insuline administrée dans chaque cas.

Les composantes de chaque patron d'entrée ont subi avant d'être présentées au réseau une transformation de type affine de manière à être comprises entre -1 et +1.

La composante du patron de sortie imposée au réseau subit également une transformation de type affine de manière à être comprise entre -1 et +1.

Chaque réseau de neurones est entraîné par une méthode de rétropropagation du gradient de l'erreur sans second ordre et sans momentum.

Le nombre d'itérations est de l'ordre de 10000 avec une adaptation séquentielle des poids.

Au terme de l'apprentissage, les paramètres liés à chaque réseau de neurones D_{1,2},...,D_{10,2}, D_{1,3},..., D_{10,3} sont mémorisés sous forme de tableaux de valeurs dans une mémoire morte qui peut être lue par le microcontrôleur 10.

Pour calculer un débit d'insuline à préconiser, le microcontrôleur 10 charge les paramètres correspondant au réseau de neurones à utiliser, qui est sélectionné en fonction de la tranche horaire considérée, comme expliqué plus haut.

Le dispositif 1 comporte en outre avantageusement au moins un réseau de neurones du type auto-associateur, connu en soi, pour obtenir une information concernant la vraisemblance des valeurs des composantes du patron d'entrée utilisé pour le calcul du débit d'insuline préconisé.

Plus particulièrement, dans l'exemple de réalisation décrit, on associe un réseau de neurones du type auto-associateur à chaque réseau de neurones D_{1,2}, D_{2,2},..., D_{10,2}, D_{1,3},..., D_{10,3}.

On a référencé sur la figure 2 Q_{1,2},..., Q_{10,2}, Q_{1,3}, ..., Q_{10,3} les réseaux de neurones du type auto-associateur respectivement associés aux réseaux de neurones D_{1,2},..., D_{10,2}, D_{1,3},... D_{10,3}.

Chaque réseau de neurones Q_{1,2}, ..., Q_{10,2}, Q_{1,3}, ..., Q_{10,3} a été entraîné pour reproduire en sortie ce qu'il reçoit en entrée et l'on peut déduire d'un écart entre la sortie et l'entrée d'un tel réseau de neurones une information susceptible d'indiquer que les valeurs des composantes du patron d'entrée sont entachées d'une erreur.

On peut alors émettre un signal d'alarme sonore et afficher un message à l'écran invitant l'utilisateur à vérifier les données entrées. En variante, on peut utiliser des réseaux de neurones du type RBF, connu en soi, pour fournir une sortie nulle si l'entrée s'écarte trop de données apprises.

Il peut arriver que lors d'un traitement, des mesures de glycémie viennent à manquer, ce qui peut gêner le calcul du débit d'insuline préconisé qui s'effectue, notamment pour la troisième phase du traitement, en fonction de plusieurs mesures antérieures de la glycémie.

C'est le cas par exemple si des mesures de la glycémie ne peuvent être effectuées suite au transfert du patient d'un service à un autre.

Les valeurs manquantes de la glycémie peuvent être fournies au dispositif par l'équipe médicale ou, dans une réalisation particulière du dispositif, être déterminées par plusieurs réseaux de neurones spécialement entraînés à fournir en sortie une valeur de la glycémie pour un certain nombre de paramètres d'entrée.

Le fonctionnement du dispositif 1 est le suivant.

Après mise en place de toute nouvelle cartouche d'insuline dans le dispositif d'injection 7, une purge du cathéter destiné à relier le dispositif d'injection 7 au patient est effectuée.

Avant chaque utilisation du dispositif 1, il est nécessaire de configurer celui-ci en rentrant un certain nombre de paramètres propres au patient considéré.

Cette configuration initiale peut s'effectuer manuellement à l'aide du clavier 4, ou bien par introduction d'une carte à puce pré-programmée dans le lecteur enregistreur 5.

La rentrée des informations au clavier 4 s'effectue dans l'exemple décrit selon un système de menu déroulant, le passage d'un menu à l'autre ou l'incrémentation et la décrémentation d'une valeur ou d'une lettre s'effectuant à l'aide de deux touches, tandis que deux autres touches permettent le passage d'un champ de données à un autre et qu'une cinquième touche permet la validation d'une entrée sélectionnée.

Suite à chaque mesure de glycémie, on rentre dans le dispositif 1 en plus de la valeur de la glycémie mesurée éventuellement la valeur de certains paramètres, dont le nombre et la nature dépendent de la phase de traitement en cours.

Après la rentrée des données demandées, le dispositif 1 calcule et affiche un débit d'insuline préconisé.

Si l'utilisateur valide la valeur affichée, l'unité centrale 2 commande le dispositif d'injection 7 pour administrer au patient la dose d'insuline correspondante.

Le dispositif 1 est également susceptible d'afficher différents messages concernant des données anormales par exemple.

Le dispositif 1 mémorise avantageusement l'ensemble des paramètres liés au traitement d'un patient sur une carte à puce par l'intermédiaire du lecteur-enregistreur 5.

On peut ainsi facilement constituer un ensemble de données permettant d'accroître le nombre de cas expérimentaux et/ou cliniques utilisés pour l'apprentissage des réseaux de neurones ou se servir des données ainsi mémorisées sur la carte à puce pour reprogrammer immédiatement, si le dispositif 1 est défectueux, un autre dispositif fonctionnant correctement.

Bien que dans l'exemple de réalisation décrit, la glycémie soit mesurée de manière discontinue, on peut, sans sortir du cadre de l'invention, effectuer une mesure continue de la glycémie pour améliorer la précision de la prédiction de la dose d'insuline à administrer pour atteindre une glycémie cible.

Dans ce cas, on peut avantageusement augmenter le nombre de tranches horaires considérées pour le calcul du débit d'insuline préconisé et augmenter conjointement le nombre de réseaux de neurones associés.

On peut encore augmenter, sans sortir du cadre de l'invention, le nombre de paramètres pris en considération pour calculer le débit d'insuline préconisé, par exemple le type de diabète affectant le patient.

Bien entendu, l'invention n'est pas limitée à l'exemple de réalisation qui vient d'être décrit.

On peut notamment utiliser un dispositif selon l'invention pour administrer à un patient une substance autre que de l'insuline en vue de réguler un état physiologique autre que la glycémie, en entraînant à partir de cas expérimentaux et/ou cliniques un ou plusieurs réseaux de neurones à fournir une indication sur la quantité de cette substance à administrer pour se rapprocher d'un état physiologique cible.

## Revendications

1. Dispositif pour déterminer la quantité d'une substance à administrer à un patient en vue de modifier un état physiologique de ce dernier, susceptible de varier en réponse à l'administration de ladite substance, et se rapprocher d'un état physiologique cible, caractérisé en ce qu'il comporte plusieurs réseaux de neurones (D_{1,2},D_{2,2},...,D_{N,2},D_{1,3},D_{2,3},...D_{N,3}) ayant été entraînés, à partir d'un ensemble de données représentatives de cas expérimentaux et/ou cliniques, pour fournir une indication de la quantité préconisée de ladite substance à administrer en fonction dudit état physiologique cible, de paramètres propres au patient considéré et de la connaissance d'au moins un état physiologique antérieur de ce dernier, chaque réseau de neurones étant en outre spécifique à une période prédéterminée du traitement du patient.

2. Dispositif selon la revendication 1, caractérisé par le fait que ledit état physiologique est la glycémie et ladite substance à administrer est de l'insuline ou un produit équivalent.

3. Dispositif selon la revendication 2, caractérisé par le fait que chaque réseau de neurones est entraîné en présentant au réseau une succession de patrons d'entrée comportant chacun parmi ses composantes une valeur représentative de la glycémie mesurée dans un cas expérimental et/ou clinique et en imposant au réseau pour chaque cas comme patron de sortie une valeur représentative du débit d'insuline administrée.

4. Dispositif selon la revendication 3, un nombre N de mesures de la glycémie du patient étant effectuées chaque jour, caractérisé par le fait qu'il comporte un premier ensemble (D_{1,2},D_{2,2},...D_{N,2}) de réseaux de neurones utilisés pour préconiser un débit d'insuline à administrer durant chaque période s'étendant entre deux mesures consécutives à partir de la deuxième mesure de glycémie jusqu'à la Nième mesure de glycémie, et un deuxième ensemble (D_{1,3},D_{2,3},..., D_{N,3}) de réseaux de neurones utilisés pour préconiser un débit d'insuline à administrer pour chaque période ultérieure s'étendant entre deux mesures consécutives de la glycémie.

5. Dispositif selon la revendication 4, caractérisé par le fait que le nombre N de mesures de glycémie effectuées chaque jour est égal à 10.

6. Dispositif selon l'une des revendications 4 et 5, caractérisé par le fait que le patron d'entrée présenté à chaque réseau de neurones dudit premier ensemble (D_{1,2},D_{2,2},...,D_{10,2}) a pour composantes :
- la dernière mesure de glycémie,
- l'avant-dernière mesure de glycémie,
- un indicateur booléen concernant une prise de sucre antérieure,
- un indicateur booléen concernant une injection antérieure ponctuelle d'insuline,
- le débit d'insuline administrée après l'avant-dernière mesure de glycémie,
- la valeur moyenne de tous les débits d'insuline depuis le début du traitement à l'exception des débits d'insuline administrée lors des périodes prandiales, jusqu'à l'avant-dernière mesure de glycémie,
- le rapport du poids du patient au carré de sa taille,
- son âge,
- son poids.

7. Dispositif selon l'une quelconque des revendications 4 à 6, caractérisé par le fait que le patron d'entrée présenté à chaque réseau de neurones dudit deuxième ensemble (D_{1,3},D_{2,3},...,D_{10,3}) de réseaux de neurones a pour composantes :
- la dernière mesure de glycémie,
- l'avant-dernière mesure de glycémie,
- l'antépénultième mesure de glycémie,
- un indicateur booléen concernant une prise de sucre antérieure,
- un indicateur booléen concernant une injection antérieure ponctuelle d'insuline,
- la glycémie mesurée la veille à la même heure,
- la glycémie mesurée la veille lors de la mesure suivant celle effectuée la veille à la même heure,
- un coefficient d'insulino-résistance
- le débit d'insuline de la veille à la même heure,
- la valeur moyenne de tous les débits d'insuline depuis le début du traitement à l'exception des débits pendant les périodes prandiales,
- le précédent débit d'insuline, et
- l'antépénultien débit d'insuline.

8. Dispositif selon l'une quelconque des revendications précédentes, caractérisé par le fait que le débit d'insuline à préconiser en réponse à la première mesure de glycémie est déterminé par une combinaison linéaire des variables suivantes :
- le rapport du poids au carré de la taille,
- la glycémie mesurée,
- l'âge,
- le poids, et si la première mesure de glycémie tombe hors d'une période prandiale,
- la glycémie cible

9. Dispositif selon la revendication 2, caractérisé par le fait que les réseaux de neurones ont été entraînés en présentant à chaque réseau une succession de patrons d'entrée comportant chacun parmi ses composantes une valeur représentative du débit d'insuline administrée dans un cas expérimental et/ou clinique et en imposant au réseau pour chaque cas comme patron de sortie une valeur représentative de la glycémie mesurée ayant conduit à l'administration dudit débit d'insuline.

10. Dispositif selon l'une quelconque des revendications précédentes, caractérisé par le fait que lesdits réseaux de neurones sont chacun du type perceptron multicouche.

11. Dispositif selon la revendication précédente, caractérisé par le fait que lesdits réseaux de neurones comportent chacun une couche cachée.

12. Dispositif selon l'une quelconque des revendications précédentes, caractérisé par le fait qu'il comporte en outre au moins un réseau de neurones supplémentaire du type auto-associateur (Q_{1,2},Q_{2,2},...,Q_{10,2},Q_{1,3},Q_{2,3},...,Q_{10,3}) entraîné à délivrer une information représentative de la vraisemblance des valeurs des composantes du patron d'entrée présenté à chaque réseau de neurones entraîné pour fournir une indication de la quantité de ladite substance à administrer.

13. Dispositif selon l'une quelconque des revendications précédentes, caractérisé par le fait qu'il comporte un dispositif d'injection (7).
